# EUROPEAN PATENT SPECIFICATION

(11) **EP 3 693 307 B1**
(45) Date of publication and mention of the grant of the patent: **08.06.2022**
(21) Application number: 20155261.9
(22) Date of filing: 03.02.2020
(51) Int. Cl.: B65H 45/24, B65H 45/28, B65H 45/16, A47K 10/42

(54) **FOLDING MACHINE FOR FOLDING FIBRE PRODUCTS**
FALTMASCHINE ZUM FALTEN VON FASERPRODUKTEN
MACHINE DE PLIAGE POUR PLIER DES PRODUITS FIBREUX

(30) Priority: 01.02.2019 TW 108104303
(43) Date of publication of application: 12.08.2020
(73) Proprietor: CHAN LI Machinery Co., Ltd, Taoyuan City 33378 (TW)
(72) Inventor: LIU, Wen-Cheng, Taoyuan City 33378 (CN)
(74) Representative: Canzler & Bergmeier Patentanwälte Partnerschaft mbB

(56) References cited:
- CN-U- 207 450 462
- JP-A- H05 105 324
- US-A- 5 310 398
- US-A1- 2006 102 643

## Description

### FIELD OF THE INVENTION

The invention relates to a folding machine for folding fibre products and a stack of fibre products produced by the folding machine, more particularly, to a folding machine that folds the fibre products with two folding devices working together to increase the folding efficiency of fibre products with three folds.

### BACKGROUND

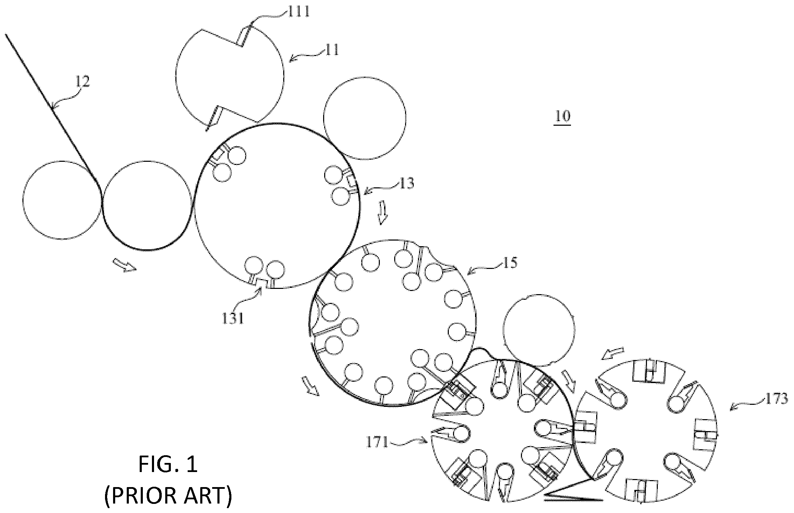

Fig. 1 is a schematic diagram of a conventional fibre-product folding device 10. The conventional folding device 10 for cutting and folding a fibre product 12 includes a cutter wheel 11, a turning wheel 13, a differential wheel 15, a first folding wheel 171, and a second folding wheel 173.

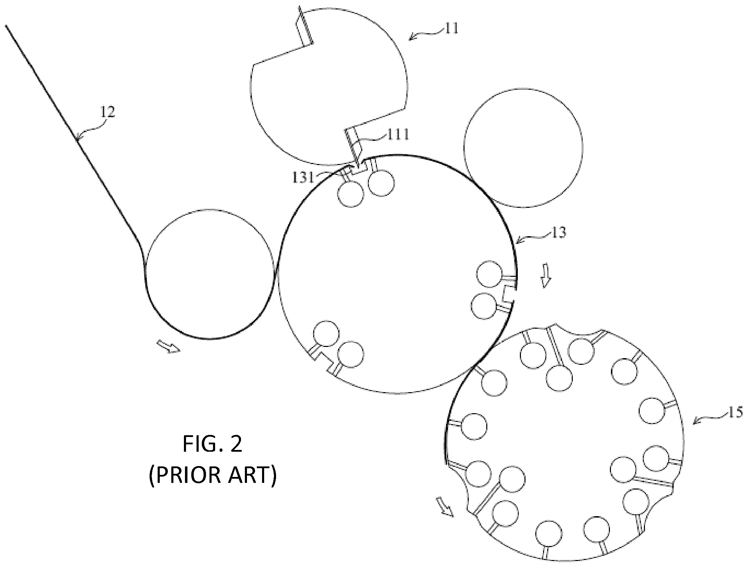

The cutter wheel 11 and the turning wheel 13 are adjacent and rotate in opposite directions; for example, the cutter wheel 11 rotates counterclockwise while the turning wheel 13 rotates clockwise. The cutter wheel has a plurality of cutters 111 disposed thereon, and the turning wheel 13 has a plurality of indentations 131 disposed on its surface. As shown in Fig. 2, during the rotation of the cutter wheel 11 and the turning wheel 13, the cutter 111 coincides with the indentation 131 and punctures the fibre product 12 at the indentation 131.

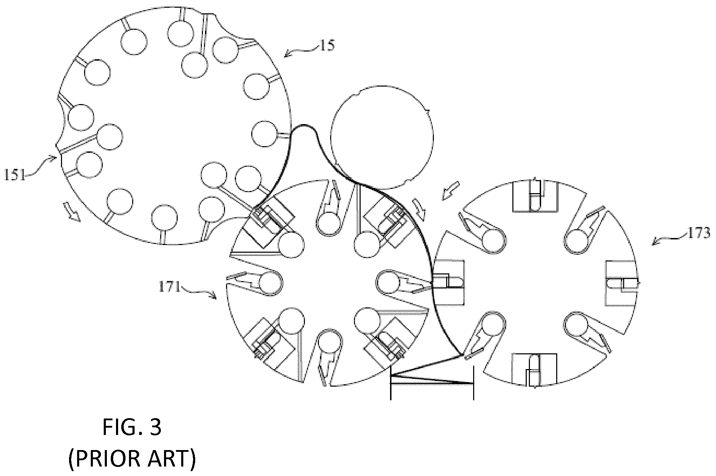

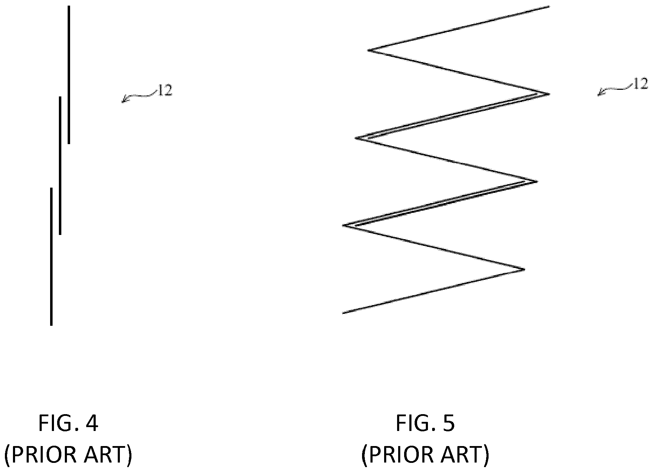

As shown in Fig. 3, the differential wheel 15 is adjacent to the turning wheel 13 and the first folding wheel 171 and receives the cut fibre product 12 from the turning wheel 13 and then transports it to the first folding wheel 171. The differential wheel 15 has a perimeter that is different in length from that of the first folding wheel 171. When the cut fibre product 12 is being transported by the differential wheel 15 to the first folding wheel 171, overlapping of the fibre product 12 occurs due to the difference in the perimeters, or the rotating speeds, of the differential wheel 15 or the first folding wheel 171. The difference in the rotating speeds may be that the rotating speed of the differential wheel 15 is faster than that of the first folding wheel 171. Subsequently, the first folding wheel 171 and the second folding wheel 173 fold the partially-overlapped fibre product 12 to produce the interfolded fibre products 12 in two folds shown in Figs. 4 and 5 that commonly seen on the market.

JP H05 105324 A discloses the preamble of claim 1 and concerns a folding machine comprising a cutting wheel, a doubling wheel, a differential wheel and a folding wheel. The cutting wheel receives and cut the paper to form a tissue paper. The doubling wheel folds the tissue paper in half, and then transmits the folded tissue paper to the differential wheel.

US 2006/102643 discloses a stack of interleaved towels is provided wherein each towel is configured from a sheet of material having a first fold offset from a centerline of the sheet to generate a first panel folded toward a first surface of the sheet and extending between the first fold and a first end of the sheet. A second fold in the sheet is made substantially parallel to the first fold to create a second panel extending between the first and second folds and a third panel extending between the second fold and a second end of the sheet such that the first end of the sheet is positioned between the second and third panels. In the stack, the third panel of each towel is disposed adjacent the first panel of an adjacent towel such that the towels are interleaved.

### SUMMARY

The invention provides a folding machine for folding fibre products as specified in claim 1.

Embodiments of the invention provide a folding machine for folding fibre products into three folds with improved folding efficiency. Conventionally, fibre products are folded by a single-sided folding device to produce fibre products with two folds, and to enhance the folding efficiency, two folding devices are sometimes disposed at two sides respectively to fold the fibre products. However, the two aforementioned folding devices are only applicable for producing fibre products with two folds, not for fibre products with three folds. Hence, a folding machine of the invention is modified to use two of the same folding machines disposed respectively at two sides that fold the fibre products at the same time and together to make fibre products into three folds, thereby effectively increasing the production efficiency for fibre products with three folds.

Embodiments of the invention provide a folding machine for folding fibre products, wherein a folding-line wheel and a platen wheel are disposed on a delivery wheel for folding fibre products carried on the delivery wheel for a first time, and then the once-folded fibre products are transported to a folding wheel to be folded for a second time, thereby forming a stack of fibre products with three folds.

Embodiments of the invention provide a folding machine for folding fibre products, wherein a plurality of folding-line wheels and a plurality of platen wheels are disposed on a delivery wheel for folding fibre products carried on the delivery wheel in half and then folding the half-folded fibre products for a first time. The once-folded fibre products are transported to a folding wheel to be folded for a second time, thereby forming a stack of double-layer fibre products with three folds.

In a folding machine according to embodiments of the invention, the perimeter of the first delivery wheel is greater than the perimeter of the first folding wheel, and the perimeter of the second delivery wheel is greater than the perimeter of the second folding wheel.

In a folding machine according to embodiments of the invention, the perimeter of the first folding wheel is two thirds of the perimeter of the first delivery wheel, and the perimeter of the second folding wheel is two thirds of the perimeter of the second delivery wheel.

In a folding machine according to embodiments of the invention, each of the first fibre product and the second fibre product includes a front end and a back end, and a direction going from the back end towards the front end is a transport direction of the first fibre product and the second fibre product. The first delivery wheel and the first folding-line wheel make the first folding line at one third of the length of the first fibre product distant from the front end, and the second delivery wheel and the second folding-line wheel make the first folding line at one third of the length of the second fibre product distant from the front end.

In a folding machine according to embodiments of the invention, the second folding line is formed by the first folding wheel and the second folding wheel and located at one third of the length of the first fibre product/the second fibre product distant from the back end.

In a folding machine according to embodiments of the invention, the first cutting device includes a first cutting knife and a first cutter wheel adjacent thereto and cuts the first fibre product that passes through between the two, and the second cutting device includes a second cutting knife and a second cutter wheel and cuts the second fibre product that passes through between the two.

A folding machine according to embodiments of the invention may further include at least one third folding-line wheel, at least one third platen wheel, at least one fourth folding-line wheel, and at least one fourth platen wheel, wherein the third folding-line wheel and the third platen wheel are disposed between the first cutting device and the first folding-line wheel and are used to fold the first fibre product in half, and the fourth folding-line wheel and the fourth platen wheel are disposed between the second cutting device and the second folding-line wheel and are used to fold the second fibre product in half.

### BRIEF DESCRIPTION OF THE DRAWINGS

The structure as well as preferred modes of use, further objects, and advantages of this invention will be best understood by referring to the following detailed description of some illustrative embodiments in conjunction with the accompanying drawings, in which:
FIG. 1 is a schematic diagram of a conventional fibre-product folding device.
FIGS. 2 and 3 are enlarged views of some components of the conventional fibre-product folding device of FIG 1.
FIG. 4 and 5 are schematic diagrams of fibre products folded and stacked by a conventional fibre-product folding device such as the fibre product folding device of FIG. 1.
FIG. 6 is a schematic diagram of a folding machine for folding fibre products according to an embodiment of the invention.
FIGS. 7-12 are enlarged views of some components of the folding machine for folding fibre products of FIG. 6.
FIGS. 13 and 14 are schematic diagrams of fibre products folded and stacked by a folding machine such as the folding machine of FIGS 6 to 12.
FIG. 15 is a schematic diagram of a folding machine for folding fibre products according to another embodiment of the invention.
FIGS. 16 and 17 are schematic diagrams of fibre products folded and stacked by a folding machine such as the folding machine of FIG. 15.

### DETAILED DESCRIPTION

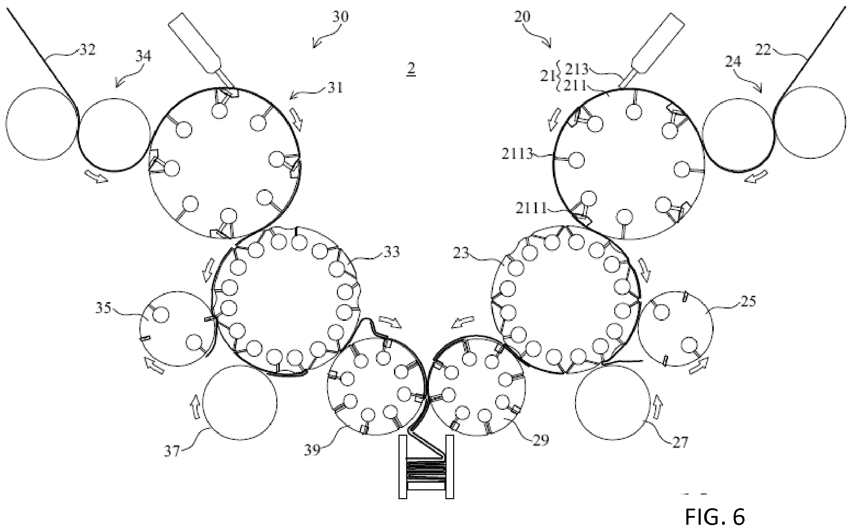

Fig. 6 is a schematic diagram of a folding machine 2 for folding fibre products. The folding machine 2 is capable of folding fibre products 22, 32, such as toilet paper, tissue paper, or paper towel, into three folded sections. The folding machine 2 includes a first folding device 20 and a second folding device 30. The first folding device 20 and the second folding device 30 are respectively used to make two folding lines on each of the fibre products 22, 32 and to fold the fibre products 22, 32 along the respective pairs of folding lines.

The first folding device 20 includes a first cutting device 21, a first delivery wheel 23, a first folding-line wheel 25, a first platen wheel 27 and a first folding wheel 29. In use, the first cutting device 21 cuts first fibre product 22 received from a first transport wheel set 24.

The first cutting device 21 may include a first cutter wheel 211 and a first cutting knife 213. The first cutter wheel 211 may include at least one cutter 2111. When the first cutter wheel 211 rotates relative to the first cutting knife 213, the at least one cutter 2111 on the first cutter wheel 211 periodically comes in contact with the first cutting knife 213 and cuts the first fibre product 22 passing between them. A cut first fibre product 22 with fixed length is formed thereby.

The first cutter wheel 211 may further include at least one suction hole 2113 connected with a source of negative pressure. Thus, the first fibre product 22 transported by the first transport wheel set 24 is drawn onto the surface of the first cutter wheel 211 by a negative pressure, making it easier for the first cutter wheel 211 and the first cutting knife 213 to cut the first fibre product 22.

The first delivery wheel 23 is disposed downstream of the first cutting device 21 and receives the cut first fibre product 22 from the first cutting device 21. In one embodiment, the first delivery wheel 23 and the first cutter wheel 211 are adjacent one another and the first delivery wheel 23 receives the cut first fibre product 22 from the first cutter wheel 211.

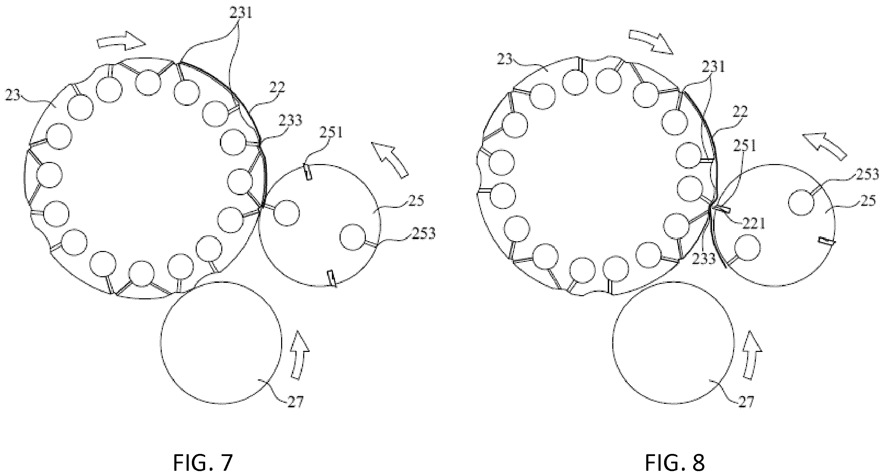

A best seen in Fig. 7, the first delivery wheel 23 includes at least one suction hole 231 connected with a source of negative pressure so that the cut first fibre product 22 carried on the first delivery wheel 23 is held against the surface of the wheel by suction. When the suction hole 231 of the first delivery wheel 23 faces the suction hole 2113 of the first cutter wheel 211, a source of negative pressure is connected with the suction hole 231 of the first delivery wheel 23, but not with the suction hole 2113 of the first cutter wheel 211, allowing the cut first fibre product 22 to be sucked into engagement with the surface of the first delivery wheel by the negative pressure at the suction hole 231 so that the cut first fibre product can be reliably transferred from the first cutter wheel to the first delivery wheel.

As shown in Figs. 8, 13 and 14, the first folding-line wheel 25 and the first delivery wheel 23 are adjacent one another and are configured to make a first folding line 221 on the cut first fibre product 22 passing between them. The first delivery wheel 23 includes at least one indentation 233 and the first folding-line wheel includes at least one protrusion 251 and at least one suction hole 253.

When the first delivery wheel 23 and the first folding-line wheel 25 rotate in different directions, for example, the first delivery wheel 23 rotates clockwise and the first folding-line wheel rotates counterclockwise, a suction hole 253 of the first folding-line wheel will periodically face a suction hole 231 of the first delivery wheel 23. At this time, a source of negative pressure is connected with the suction hole 253 of the first folding-line wheel 25, but not with the facing suction hole 231 of the first delivery wheel 23, so that one end of the cut first fibre product 22 is drawn against the surface of the first folding-line wheel 25 by the negative pressure at the suction hole 253.

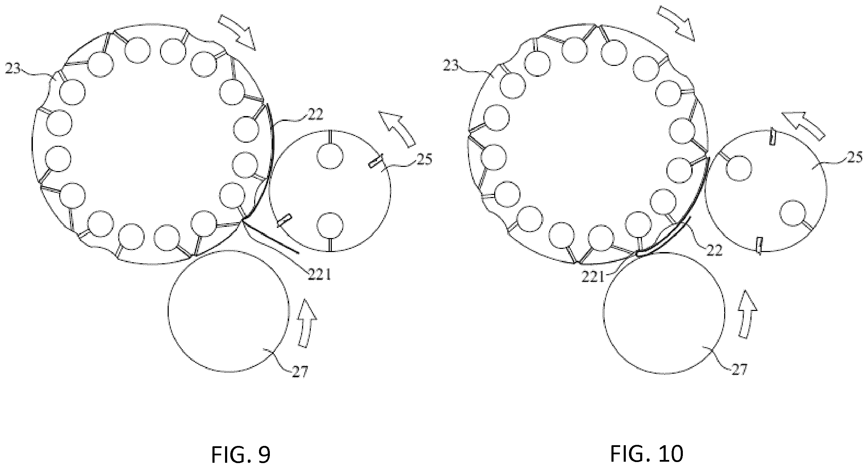

Referring to Fig. 8, as the first folding-line wheel 25 and the first delivery wheel 23 continue to rotate, a protrusion 251 of the first folding-line wheel 25 periodically meets an indentation 233 of the first delivery wheel 23 and as the cut first fibre product 22 passes between them, a first folding line 221 (Fig. 9) is formed.

Referring to FIG. 9, after the first folding line 221 has been formed, the supply of negative pressure to the suction hole 253 of the first folding-line wheel 25 is stopped and the cut first fibre product 22 is released. At the same time, the first delivery wheel 23 continues to transport the first fibre product 22 and the first fibre product 22 forms a pre-fold, or partially folded, status along the first folding line 221 as shown in Fig. 9.

The first platen wheel 27 is disposed downstream of the first folding-line wheel 25 and adjacent to the first delivery wheel 23. The first platen wheel 27 can be a smooth-surfaced circular column, or roller. As shown in FIG. 10, with continuing rotation of the first delivery wheel 23, the cut first fibre product 22 in the pre-fold status is transported to the first platen wheel 27. As the cut first fibre product 22 passes between the first platen wheel 27 and the first delivery wheel 23, it is pressed along the first folding line 221. This folds the cut first fibre product 22 for a first time along the first folding line 221.

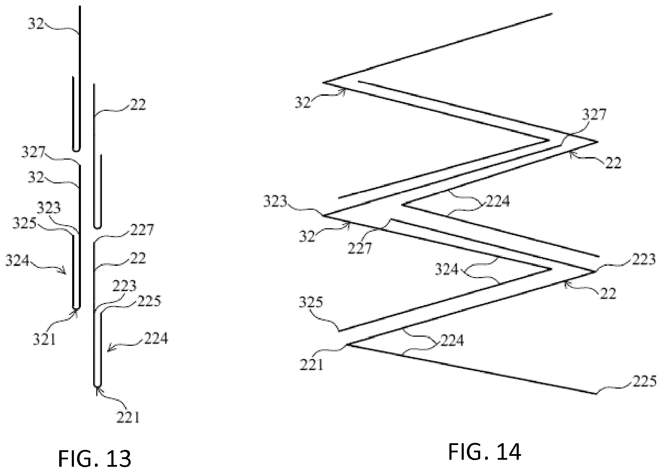

Referring to Figs. 13 and 14, the respective ends of the cut first fibre product 22 can be defined as a front (leading) end 225 and a back (trailing) end 227, according to the transport direction of the first fibre product 22, wherein the direction going from the back end 227 towards the front end 225 is the transport direction. The distance between the first folding line 221 and the front end 225 is about one third of the length of the cut first fibre product 22. A first fold section 224 is formed between the front end 225 and the first folding line 221 after the first fibre product 22 is folded along the first folding line 221 to produce a once-folded first fibre product 22.

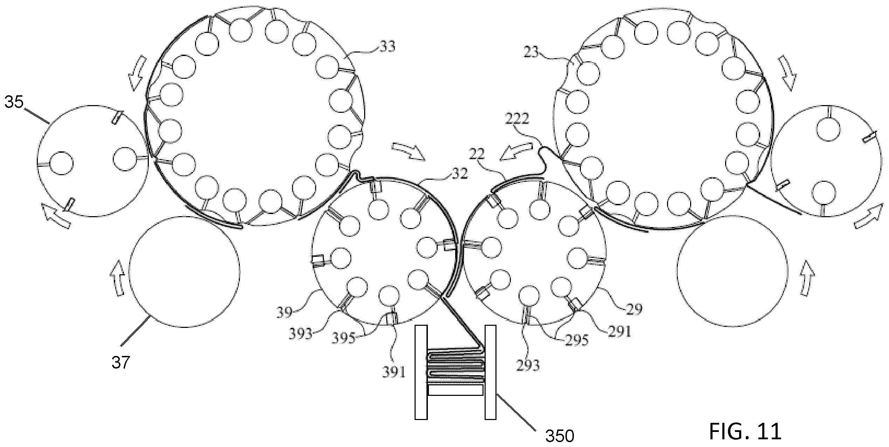

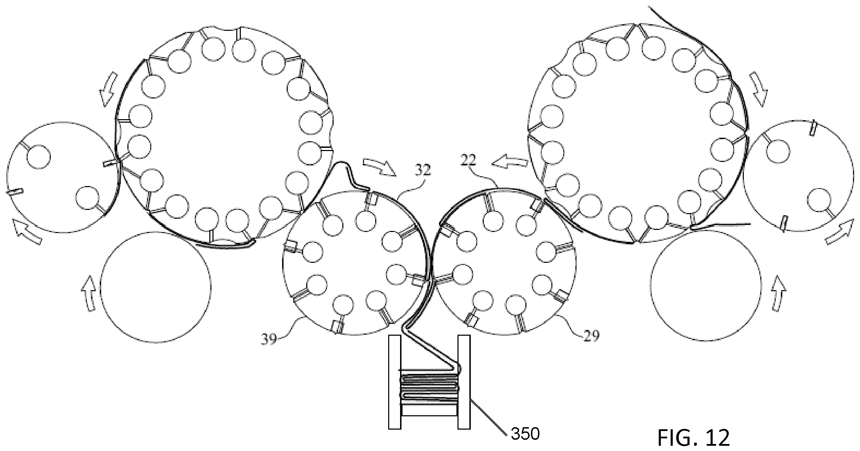

Referring to Figs. 11 and 12, the first folding wheel 29 is adjacent the first delivery wheel 23 and receives threrefrom the once-folded first fibre product 22. The length of the first fibre product 22 that has been folded for a first time is smaller than the length of an unfolded cut first fibre product 22. In other words, the length of the cut first fibre product 22 received by the first delivery wheel 23 from the first cutting device 21 is greater than the length of the once-folded first fibre product 22 received by the folding wheel 29 from the first delivery wheel 23. Therefore the perimeter, or circumference, of the first folding wheel 29 is smaller than the perimeter, or circumference, of the first delivery wheel 23.

Referring to Figs. 11 and 12, the first folding wheel 29 includes at least one protrusion 291, at least one indentation 293 and at least one suction hole 295. The protrusion 291 and the indentation 293 are disposed one behind the other on the surface of the first folding wheel 29 and when multiple protrusions and indentations are provided, as in the illustrated embodiment, they are disposed in an alternating arrangement in which a protrusion is followed by an indentation and vice versa. A respective suction hole 295 is disposed inside or near the or each protrusion 291 and the indentation 293, so that the once-folded first fibre product 22 can be pulled against a protrusion 291 and/or indentation 293 by an applied suction force.

In embodiments in which the folding machine 2 produces the fibre product 22 with three folded sections, the first folding-line wheel 25 and the first delivery wheel 23 make a first folding line 221 that is spaced about one third of the length of the cut first fibre product 22 from the front end 225 and the first platen wheel 27 and the first delivery wheel 23 fold the first fibre product 22 for a first time along the first folding line 221.

The length of the first fibre product 22 that has been folded for a first time is about two thirds of its original length and the perimeter, or circumference, of the first folding wheel 29 is two thirds of the perimeter, or circumference, of the first delivery wheel 23. Thus, the unfolded first fibre product 22 is held smoothly against the surface of the first delivery wheel 23 and the once-folded first fibre product 22 is also held smoothly against the surface of the first folding wheel 29.

As shown in Fig, 11, during the transfer of the once-folded first fibre product 22 to the first folding wheel 29 by the first delivery wheel 23, one end of the first fibre product 22 is held by the first folding wheel 29 and the first fibre product 22 is rotated thereby, whilst the other end of the first fibre product 22 is still held by the first delivery wheel 23.

As shown in Figs, 11 and 12, since the perimeter of the first delivery wheel 23 is greater than that of the first folding wheel 29, when the once-folded first fibre product 22 is transferred to the first folding wheel 29 by the first delivery wheel 23, the part of the first fibre product 22 between the first delivery wheel 23 and the first folding wheel 29 forms a bent, or semi-folded part 222. The bent, or semi-folded, part 222 may have an arcuate or arched form.

It is to be noted that although the bent, or semi-folded, part 222 formed during the transfer of the first fibre product 22 between the first delivery wheel 23 and the first folding wheel 29 seems similar to the bent portion produced by the prior art folding machine shown in Fig. 1, the bent, or semi-folded part 222 does not cover over a next first fibre product 22 when the first delivery wheel 23 releases the first fibre product 22. Thus, the adjacent or successive first fibre products 22 on the first folding wheel 29 do not overlap one another.

Referring to Fig. 6, the second folding device 30 includes a second cutting device 31, a second delivery wheel 33, a second folding-line wheel 35, a second platen wheel 37, and a second folding wheel 39. In use, the second cutting device 31 receives the second fibre product 32 from a second transport wheel set 34 and cuts the second fibre product 32.

The second delivery wheel 33 receives the cut second fibre product 32 from the second cutting device 31 and in cooperation with the second folding-line wheel 35 forms a first folding line 321 on the cut second fibre product 32. The second delivery wheel 33 and the second platen wheel 37 fold the cut second fibre product 32 for a first time along the first folding line 321 and transport the once-folded second fibre product 32 to the second folding wheel 39. The configuration and operation of the second folding device 30 is similar to that of the first folding device 20. Accordingly, a full description of its features and operation will not be repeated herein.

Although the configuration of the second folding device 30 is similar to that of the first folding device 20, their operations are not in synchronised. In particular, while the speed of rotation of the first folding wheel 29 and the second folding wheel 39 is the same, there is a phase difference between the two folding wheels 29, 39 so as to allow folding of the first fibre product 22 and the second fibre product 32 for a second time.

Referring to Figs. 13 and 14, the ends of the second fibre product 32 may be respectively defined as a front (leading) end 325 and a back (trailing) end 327 according to a transport direction of the second fibre product 22, wherein the direction from the back end 327 towards the front end 325 is the transport direction. The first folding line 321 formed on the second fibre product 32 is spaced about one third of the length of the second fibre product 32 from the front end 325 and a first fold section 324 is formed after the cut second fibre product 32 is folded along the first folding line 321.

The once-folded first fibre product 22 and the once-folded fibre product 32 are sequentially transported to the first folding wheel 29 and the second folding wheel 39. The first folding wheel 29 and the second folding wheel 39 are adjacent and configured to fold the first fibre product 22 and the second fibre product 32 as they pass through between the two folding wheels 29, 39 into a folding station 350.

As shown in FIG. 11, the second folding wheel 39 includes at least one protrusion 391, at least one indentation 393 and at least one suction hole 395. The protrusion 391 and the indentation 393 are disposed one behind the other on the surface of the second folding wheel 39 and in embodiments with multiple protrusions and indentations as shown in the drawings, the protrusions and indentations are disposed in an alternating arrangement so that an indentation follows a protrusion and vice versa. A suction hole 395 is disposed inside or near the or each protrusion 391 and indentation 393, so that the second fibre product 32 can be held in engagement with the protrusion 391 and/or the indentation 393 by an applied negative pressure, or suction, force.

The first folding wheel 29 and the second folding wheel 39 are configured to make a second folding line 223, 323 on the first and second fibre products 22, 32. The second folding lines 223, 323 are at about where the respective front ends 225, 325 overlap the respective fibre products 22, 32. Then the first and second fibre products 22, 32 are folded for a second time along the second folding line 223, 323, thereby forming a fibre products with two folds that define three folded sections.

The or each protrusion 291 and indentation 293 of the first folding wheel 29 and the or each indentation 393 and protrusion 391 of the second folding wheel 39 are configured to periodically meet protrusion to indentation as the first and second folding wheels contra rotate. When the once-folded first fibre product 22 is transported by the first folding wheel 29 to and between the first and second folding wheels 29, 39, as a protrusion 391 of the second folding wheel 39 meets an indentation 293 of the first folding wheel 29, a second folding line 223 is formed on the once-folded first fibre product 22 and subsequently the once-folded first fibre product 22 is folded for a second time along the second folding line 223. Moreover, as shown in Fig. 11, the second fibre product 32 transported by the second folding wheel 39 overlaps the first fold section 224 of the first fibre product 22 transported by the first folding wheel 29.

As shown in Fig. 12, the first fibre product 22 transported by the first folding wheel 29 also overlaps the first fold section 324 of the second folding product 32 transported by the second folding wheel 39 so the first fibre product 22 and the second fibre product 32 adjacent to one another are alternately stacked to form an interfolded tissue paper with three fold sections.

Figs. 13 and 14 are schematic diagrams illustrating a stack of fibre products produced and stacked by the folding machine 2. The stack of fibre products includes a plurality of first fibre products 22 and a plurality of second fibre products 32 with adjacent, or successive, first and second fibre products 22 and 32 overlapped in partial regions and thus form the stack of interfolded fibre products, for example, a stack of interfolde tissue paper.

The first fibre product 22 includes a front (leading) end 225, a back (trailing) end 227, a first folding line 221, a second folding line 223, and a first fold section 224, wherein a direction going from the back end 227 towards the front end 225 is the transport direction of the first fibre product 22. The distance between the first folding line 221 and the front end 225 is about one third of the length of the first fibre product 22, wherein the first fibre product 22 is folded along the first folding line 221 and forms the first fold section 224. The distance between the second folding line 223 and the back end 227 is about one third of the length of the first fibre product 22, wherein the second folding line 223 and the front end 225 overlap in location. Thus, when folded along the first and second folding lines 221, 223, the first fibre product may be folded into three equi-length section, including the first folded section 224.

The second fibre product 32 includes a front (leading) end 325, a back (trailing) end 327, a first folding line 321, a second folding line 323, and a first fold section 324, wherein a direction going from the back end 327 towards the front end 325 is the transport direction of the second fibre product 32. The distance between the first folding line 321 and the front end 325 is about one third of the length of the second fibre product 32, wherein the second fibre product 32 is folded along the first folding line 321 and forms the first fold section 324. The distance between the second folding line 323 and the back end 327 is about one third of the length of the second fibre product 32, wherein the second folding line 323 and the front end 325 overlap in location. Thus, when folded along the first and second folding lines 321, 323, the second fibre product may be folded into three equi-length section, including the first fold section 324.

As shown in FIG. 14, the first fibre product 22 and the second fibre product 32 are alternately arranged and stacked, wherein the first fold section 224 of the folded first fibre products 22 is covered by the preceding second fibre product 32, for example, covered by a region between the back end 327 and the second folding line 323 of the second fibre product 32, and the first fold section 324 of the second fibre products 32 is covered by the preceding first fibre product 22, for example, covered by a region between the back end 227 and the second folding line 223 of the first fibre product 22.

Moreover, the region of the first fibre product 22 between its back end 227 and its second folding line 223 is covered by a region of the next following first fibre product 22 between its front end 225 and its first folding line 221, wherein the region of the first fibre products 22 between their back end 227 and their second folding line 223 is located between the next following second fibre product 32 and the next following first fibre product 22, and the region of the second fibre products 32 between their back end 327 and their second folding line 323 is located between the next following first fibre product 22 and the next following second fibre product 32.

For the convenience of description, the fibre products are referenced as first fibre products 22 and second fibre products 32. In practical applications, the first fibre products 22 and the second fibre products 32 may be made of the same material and have the same size and thickness.

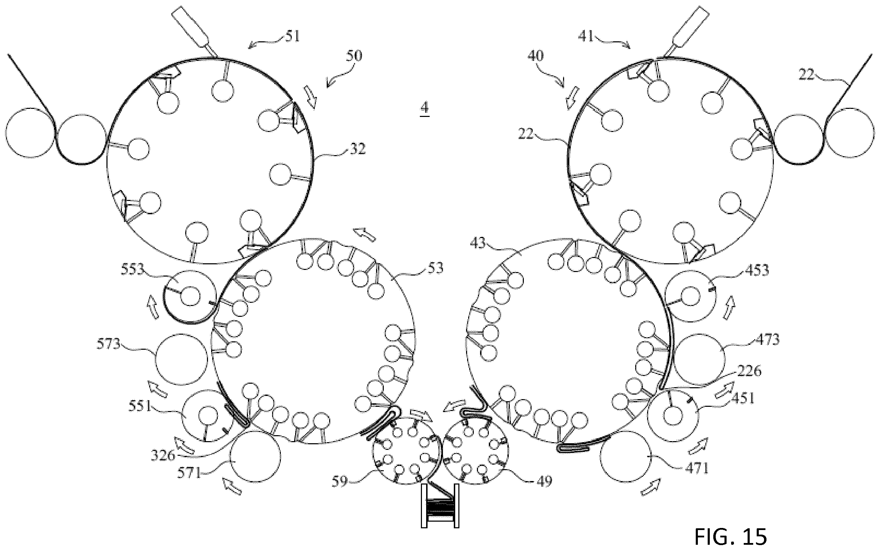

Fig. 15 is a schematic diagram illustrating another folding machine 4 for folding fibre products. The folding machine 4 includes a first folding device 40 and a second folding device 50 for folding respective fibre products 22, 32 passing through the two folding devices 40, 50.

The first folding device 40 includes a first cutting device 41, a first delivery wheel 43, a first folding-line wheel 451, a first platen wheel 471, a third folding-line wheel 453, a third platen wheel 473, and a first folding wheel 49. The second folding device 50 includes a second cutting device 51, a second delivery wheel 53, a second folding-line wheel 551, a second platen wheel 571, a fourth folding-line wheel 553, a fourth platen wheel 573, and a second folding wheel 59.

The folding machine 4 is similar to the folding machine 2 and differs in that the folding machine 4 further includes the third folding-line wheel 453, the third platen wheel 473, the fourth folding-line wheel 553, and the fourth platen wheel 573. The third folding-line wheel 453 and the third platen wheel 473 are adjacent to the first delivery wheel 43 and disposed between the first folding-line wheel 451 and the first cutting device 41. The fourth folding-line wheel 553 and the fourth platen wheel 573 are adjacent to the second delivery wheel 53 and disposed between the second folding-line wheel 551 and the second cutting device 51.

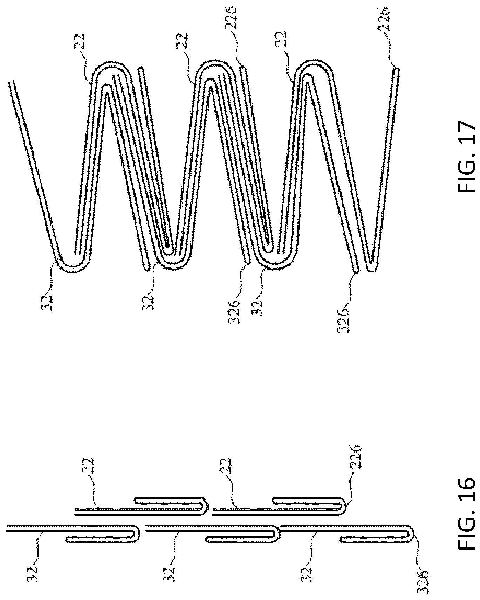

The first folding device 40 cuts the received first fibre products 22 with the first cutting device 41 and thus cut first fibre products 22 with a fixed length are formed. The first delivery wheel 43 receives the cut first fibre product 22 from the first cutting device 41 and the third folding-line wheel 453 is configured to makes a centre folding line 226 (FIG. 16) approximately halfway along the length of the cut first fibre product 22 carried by the first delivery wheel 43. The centre folding 226 may extend perpendicular to the longitudinal axis of the cut first fibre product.

The third platen wheel 473 is disposed downstream of the third folding-line wheel 453. The third platen wheel 473 and the first delivery wheel 43 are cooperable to press the first fibre product 22 passing between them along the centre folding line 226 to fold the first fibre product 22 in half. Thus, a double thickness first fibre product having a length substantially half the length of the cut first fibre product is formed.

The first fibre product 22 that has been folded in half is transported to the first folding-line wheel 451 and the first platen wheel 471 to be folded again at one third of its length. The first folding wheel 451 and the first platen wheel 471 of this embodiment fold the first fibre product 22 in a similar way to that of the first folding wheel 25 and the first platen wheel 27 in the previous embodiment, and the difference is that the first fibre product 22 being folded by the first folding wheel 451 and the first platen wheel 471 in this embodiment had been folded in half.

The configuration and operation of the second folding device 50 is similar to that of the first folding device 40, wherein the fourth folding-line wheel 553 makes a centre folding line 326 (FIG. 16) on a centre line or a bisecting line of the second fibre product 32 carried by the second delivery wheel 53. The centre folding line 326 may extend perpendicular to the longitudinal axis of the cut second fibre product. The fourth platen wheel 573 and the second delivery wheel 53 fold the second fibre product 32 in half along the center folding line 326, and then the second folding-line wheel 551 and the second platen wheel 571 fold the second fibre product 32 that has been folded in half at one third of its length.

The first folding wheel 49 and the second folding wheel 59 also operate similarly to the first folding wheel 29 and the second folding wheel 39 in the previous embodiment and hence will not be repeated herein.

Furthermore, in the folding machine 2, the perimeter of the first folding wheel 29 can be two thirds of the perimeter of the first delivery wheel 23, and the perimeter of the second folding wheel 39 is two thirds of the perimeter of the second delivery wheel 33. However, for the folding machine 4 of this embodiment, the perimeter of the first folding wheel 49 can be one third of the perimeter of the first delivery wheel 43, and the perimeter of the second folding wheel 59 is one third of the perimeter of the second delivery wheel 53.

The fibre products folded and stacked by the folding machine 4 of this embodiment are substantially the same as those folded and stacked by the folding machine 2 in the previous embodiment. As shown in Figs. 16 and 17, the difference is that the first fibre product 22 and the second fibre product 32 produced by the folding machine 4 of this embodiment have a double-layer structure from being folded in half and are thicker in thickness.

The folding lines formed by the folding machines 2, 4 extend transversely of the cut fibre products and may extend perpendicular to the longitudinal axis of the cut fibre products.

It is to be understood that the above disclosure is only of embodiments of the invention, and do not define the scope of the invention, which is limited only by the claims hereof.

## Claims

1. A folding machine for folding fibre products, comprising:
a first folding device (20; 40) comprising:
a first cutting device (21;41) for cutting a first fibre product (22);
a first delivery wheel (23; 43), the first delivery wheel arranged to receive the cut first fibre product from the first cutting device;
a first folding-line wheel (25; 451) adjacent to the first delivery wheel, wherein the first folding-line wheel and the first delivery wheel are arranged to make a first folding line (221) on the first fibre product;
a first platen wheel (27; 471) adjacent to the first delivery wheel and downstream of the first folding-line wheel, wherein the first platen wheel and the first delivery wheel are arranged to fold the first fibre product along the first folding line (221); and
a first folding wheel (29; 49) adjacent to the first delivery wheel, the first folding wheel arranged to receive the first fibre product from the first delivery wheel (23; 43); and
a second folding device (30; 50) comprising:
a second cutting device (31; 51) for cutting a second fibre product (32);
a second delivery wheel (33; 53), the second delivery wheel arranged to receive the cut second fibre product from the second cutting device;
a second folding-line wheel (35; 351) adjacent to the second delivery wheel, wherein the second folding-line wheel and the second delivery wheel are arranged to make a first folding line (321) on the second fibre product;
a second platen wheel (37; 571) adjacent to the second delivery wheel and downstream of the second folding-line wheel, wherein the second platen wheel and the second delivery wheel arranged to fold the second fibre product along the first folding line (321); and
a second folding wheel (39; 59) adjacent to the second delivery wheel, the second folding wheel arranged to receive the second fibre product from the second delivery wheel (33; 53), wherein the first folding wheel and the second folding wheel are adjacent and arranged to make a second folding line (223; 323) on the first fibre product and the second fibre product passing therebetween and fold the first fibre product and the second fibre product along the respective second folding lines (223; 323), **characterized in that** the first delivery wheel (23; 43) and the second delivery wheel (33; 53) each comprise at least one indentation (233) and at least one suction hole (231), the first folding-line wheel (25; 451) and the second-folding line wheel (35; 551) each comprise at least one protrusion (251) and at last one suction hole (253), and the protrusions of the first folding-line wheel and the second folding-line wheel and the indentations of the first delivery wheel and the second delivery wheel are arranged such that, in use, as the wheels rotate the protrusions of the first and second-folding line wheels respectively meet the indentations of the first and second delivery wheels to make the respective first folding lines on the first fibre product carried by the first delivery wheel and on the second fibre product carried by the second delivery wheel.

2. The folding machine of claim 1, wherein a perimeter of the first delivery wheel (23; 43) is greater than a perimeter of the first folding wheel (29; 49), and a perimeter of the second delivery wheel (33; 53) is greater than a perimeter of the second folding wheel (39).

3. The folding machine of claim 2, wherein the perimeter of the first folding wheel (29; 49) is two thirds of the perimeter of the first delivery wheel (23; 43), and the perimeter of the second folding wheel is two thirds of the perimeter of the second delivery wheel (33; 59).

4. The folding machine of any one of preceding claims, wherein the first fibre product (22) and the second fibre product (32) each comprise a front end (225; 325) and a back end (227, 327), a direction going from the back end towards the front end is a delivery direction of the first fibre product and the second fibre product, the first delivery wheel (23; 43) and the first folding-line wheel (25) are arranged to make the first folding line (221) on the first fibre product at one third of the length of the first fibre product distant from the front end (227), and the second delivery wheel (33; 53) and the second folding-line wheel are arranged to make the first folding line (321) on the second fibre product at one third of the length of the second fibre product distant from the front end (325).

5. The folding machine of claim 4, wherein the first folding wheel (29; 49) and the second folding wheel (39; 53) are arranged to make the second folding line on the first fibre product and the second fibre product at one third of the length of the first fibre product and the second fibre product distant from the respective back ends (227, 372) of the first and second fibre products.

6. The folding machine of any one of the preceding claims, wherein each of the first folding wheel and the second folding wheel comprises at least one protrusion and at least one indentation, and the protrusion (291) and the indentation (293) of the first folding wheel (29) conform respectively to the indentation (393) and the protrusion (391) of the second folding wheel (39) to make the second folding line on the first fibre product and the second fibre product.

7. The folding machine of any of the preceding claims, wherein the first cutting device (21; 41) comprises a first cutting knife (213) and a first cutter wheel (211), the first cutting knife and the first cutter wheel are adjacent and arranged to cut the first fibre product passing therebetween, and the second cutting device (31; 51) comprises a second cutting knife and a second cutter wheel, the second cutting knife and the second cutter wheel are adjacent and arranged to cut the second fibre product passing therebetween.

8. The folding machine of any one of the preceding claims, further comprising at least one third folding-line wheel (453), at least one third platen wheel (473), at least one fourth folding-line wheel (553), and at least one fourth platen wheel (573), wherein the third folding-line wheel and the third platen wheel are disposed between the first cutting device (41) and the first folding-line wheel (451) for folding the first fibre product in half, and the fourth folding-line wheel and the fourth platen wheel are disposed between the second cutting device (51) and the second folding-line wheel (551) for folding the second fibre product in half.

## Patentansprüche

1. Faltmaschine zum Falten von Faserprodukten, umfassend:
eine erste Faltvorrichtung (20; 40), umfassend:
eine erste Schneidvorrichtung (21; 41) zum Schneiden eines ersten Faserprodukts (22);
ein erstes Übergaberad (23; 43), wobei das erste Übergaberad so angeordnet ist, dass es das geschnittene erste Faserprodukt von der ersten Schneidvorrichtung empfängt;
ein erstes Faltlinienrad (25; 451) benachbart zu dem ersten Übergaberad, wobei das erste Faltlinienrad und das erste Übergaberad so angeordnet sind, dass sie eine erste Faltlinie (221) auf dem ersten Faserprodukt erzeugen;
ein erstes Druckrad (27; 471), benachbart zu dem ersten Übergaberad und nachgelagert dem ersten Faltlinienrad, wobei das erste Druckrad und das erste Übergaberad so angeordnet sind, dass sie das erste Faserprodukt entlang der ersten Faltlinie (221) falten; und
ein erstes Faltrad (29; 49), benachbart zu dem ersten Übergaberad, wobei das erste Faltrad so angeordnet ist, dass es das erste Faserprodukt vom ersten Übergaberad (23; 43) empfängt;
und
eine zweite Faltvorrichtung (30; 50), umfassend:
eine zweite Schneidvorrichtung (31 ;51) zum Schneiden eines zweiten Faserprodukts (32);
ein zweites Übergaberad (33; 53), wobei das zweite Übergaberad so angeordnet ist, dass es das geschnittene zweite Faserprodukt von der zweiten Schneidvorrichtung empfängt;
ein zweites Faltlinienrad (35; 351), benachbart zu dem zweiten Übergaberad, wobei das zweite Faltlinienrad und das zweite Übergaberad so angeordnet sind, dass sie eine erste Faltlinie (321) auf dem zweiten Faserprodukt erzeugen;
ein zweites Druckrad (37; 571), benachbart zu dem zweiten Übergaberad und nachgelagert dem zweiten Faltlinienrad, wobei das zweite Druckrad und das zweite Übergaberad so angeordnet sind, dass sie das zweite Faserprodukt entlang der ersten Faltlinie (321) falten; und
ein zweites Faltrad (39; 59), benachbart zu dem zweiten Übergaberad, wobei das zweite Faltrad so angeordnet ist, dass es das zweite Faserprodukt vom zweiten Übergaberad (33; 53) empfängt,
wobei das erste Faltrad und das zweite Faltrad benachbart sind und so angeordnet sind, dass sie eine zweite Faltlinie (223; 323) auf dem ersten Faserprodukt und dem zweiten Faserprodukt erzeugen, die dazwischen verlaufen, und das erste Faserprodukt und das zweite Faserprodukt entlang den jeweiligen zweiten Faltlinien (223; 323) falten, **dadurch gekennzeichnet, dass** das erste Übergaberad (23; 43) und das zweite Übergaberad (33; 53) jeweils mindestens eine Vertiefung (233) und mindestens eine Ansaugöffnung (231) umfassen, das erste Faltlinienrad (25; 451) und das zweite Faltlinienrad (35; 551) jeweils mindestens einen Vorsprung (251) und mindestens eine Ansaugöffnung (253) umfassen, und die Vorsprünge des ersten Faltlinienrads und des zweiten Faltlinienrads und die Vertiefungen des ersten Übergaberads und des zweiten Übergaberads so angeordnet sind, dass im Gebrauch, wenn sich die Räder drehen, die Vorsprünge des ersten und des zweiten Faltlinienrads jeweils auf die Vertiefungen des ersten und des zweiten Übergaberads treffen, um die jeweiligen ersten Faltlinien auf dem durch das erste Übergaberad getragenen ersten Faserprodukt und auf dem durch das zweiten Übergaberad getragenen zweiten Faserprodukt zu erzeugen.

2. Faltmaschine nach Anspruch 1, wobei eine Umfangslänge des ersten Übergaberads (23; 43) größer ist als eine Umfangslänge des ersten Faltrads (29; 49) und eine Umfangslänge des zweiten Übergaberads (33; 53) größer ist als eine Umfangslänge zweiten Faltrads (39).

3. Faltmaschine nach Anspruch 2, wobei die Umfangslänge des ersten Faltrads (29; 49) zwei Drittel der Umfangslänge des ersten Übergaberads (23; 43) beträgt und die Umfangslänge des zweiten Faltrads zwei Drittel der Umfangslänge des zweiten Übergaberads (33; 59) beträgt.

4. Faltmaschine nach einem beliebigen der vorangehenden Ansprüche, wobei das erste Faserprodukt (22) und das zweite Faserprodukt (32) jeweils ein Vorderende (225; 325) und ein Hinterende (227, 327) umfassen, eine Richtung, die vom Hinterende zum Vorderende geht, eine Übergaberichtung des ersten Faserprodukts und des zweiten Faserprodukts ist, das erste Übergaberad (23; 43) und das erste Faltlinienrad (25) so angeordnet sind, dass sie die erste Faltlinie (221) auf dem ersten Faserprodukt bei einem Drittel der Länge des ersten Faserprodukts vom Vorderende (227) entfernt erzeugen, und das zweite Übergaberad (33; 53) und das zweite Faltlinienrad so angeordnet sind, dass sie die erste Faltlinie (321) auf dem zweiten Faserprodukt bei einem Drittel der Länge des zweiten Faserprodukts vom Vorderende (325) entfernt erzeugen.

5. Faltmaschine nach Anspruch 4, wobei das erste Faltrad (29; 49) und das zweite Faltrad (39; 53) so angeordnet sind, dass sie die zweite Faltlinie auf dem ersten Faserprodukt und dem zweiten Faserprodukt bei einem Drittel der Länge des ersten Faserprodukts und des zweiten Faserprodukts von den jeweiligen Hinterenden (227, 372) des ersten und des zweiten Faserprodukts entfernt erzeugen.

6. Faltmaschine nach einem beliebigen der vorangehenden Ansprüche, wobei jedes aus dem ersten Faltrad und dem zweiten Faltrad mindestens einen Vorsprung und mindestens eine Vertiefung umfasst und der Vorsprung (291) und die Vertiefung (293) des ersten Faltrads (29) jeweils der Vertiefung (393) und dem Vorsprung (391) des zweiten Faltrads (39) entsprechen, um die zweite Faltlinie auf dem ersten Faserprodukt und dem zweiten Faserprodukt zu erzeugen.

7. Faltmaschine nach beliebigen der vorangehenden Ansprüche, wobei die erste Schneidvorrichtung (21; 41) ein erstes Schneidmesser (213) und ein erstes Schneidrad (211) umfasst und das erste Schneidmesser und das erste Schneidrad benachbart sind und angeordnet sind, das dazwischen hindurchlaufende erste Faserprodukt abzuschneiden, und die zweite Schneidvorrichtung (31; 51) ein zweites Schneidmesser und ein zweites Schneidrad umfasst und das zweite Schneidmesser und das zweite Schneidrad benachbart sind und angeordnet sind, das dazwischen hindurchlaufende zweite Faserprodukt abzuschneiden.

8. Faltmaschine nach einem beliebigen der vorangehenden Ansprüche, weiter umfassend mindestens ein drittes Faltlinienrad (453), mindestens ein drittes Druckrad (473), mindestens ein viertes Faltlinienrad (553) und mindestens ein viertes Druckrad (573), wobei das dritte Faltlinienrad und das dritte Druckrad zwischen der ersten Schneidvorrichtung (41) und dem ersten Faltlinienrad (451) zum Falten des ersten Faserprodukts in zwei Hälften angeordnet sind, und das vierte Faltlinienrad und das vierte Druckrad zwischen der zweiten Schneidvorrichtung (51) und dem zweiten Faltlinienrad (551) zum Falten des zweiten Faserprodukts in zwei Hälften angeordnet sind.

## Revendications

1. Machine à plier pour plier des produits en fibres, comprenant :
un premier dispositif de pliage (20 ; 40) comprenant :
un premier dispositif de coupe (21 ; 41) pour couper un premier produit en fibres (22) ;
une première roue de livraison (23 ; 43), la première roue de livraison étant agencée pour recevoir le premier produit en fibres coupé depuis le premier dispositif de coupe ;
une première roue de ligne de pliage (25 ; 451) adjacente à la première roue de livraison, dans laquelle la première roue de ligne de pliage et la première roue de livraison sont agencées pour réaliser une première ligne de pliage (221) sur le premier produit en fibres ;
une première roue à platine (27 ; 471) adjacente à la première roue de livraison et en aval de la première roue de ligne de pliage, dans laquelle la première roue à platine et la première roue de livraison sont agencées pour plier le premier produit en fibres le long de la première ligne de pliage (221) ; et
une première roue de pliage (29 ; 49) adjacente à la première roue de livraison, la première roue de pliage étant agencée pour recevoir le premier produit en fibres depuis la première roue de livraison (23 ; 43) ;
et
un deuxième dispositif de pliage (30 ; 50) comprenant :
un deuxième dispositif de coupe (31 ; 51) pour couper un deuxième produit en fibres (32) ;
une deuxième roue de livraison (33 ; 53), la deuxième roue de livraison étant agencée pour recevoir le deuxième produit en fibres coupé depuis le deuxième dispositif de coupe ;
une deuxième roue de ligne de pliage (35 ; 351) adjacente à la deuxième roue de livraison, dans laquelle la deuxième roue de ligne de pliage et la deuxième roue de livraison sont agencées pour réaliser une première ligne de pliage (321) sur le deuxième produit en fibres ;
une deuxième roue à platine (37 ; 571) adjacente à la deuxième roue de livraison et en aval de la deuxième roue de ligne de pliage, dans laquelle la deuxième roue à platine et la deuxième roue de livraison sont agencées pour plier le deuxième produit en fibres le long de la première ligne de pliage (321) ; et
une deuxième roue de pliage (39 ; 59) adjacente à la deuxième roue de livraison, la deuxième roue de pliage étant agencée pour recevoir le deuxième produit en fibres depuis la deuxième roue de livraison (33 ; 53),
dans laquelle la première roue de pliage et la deuxième roue de pliage sont adjacentes et agencées pour réaliser une deuxième ligne de pliage (223 ; 323) sur le premier produit en fibres et le deuxième produit en fibres passant entre elles et plier le premier produit en fibres et le deuxième produit en fibres le long des deuxièmes lignes de pliage (223 ; 323) respectives, **caractérisée en ce que** la première roue de livraison (23 ; 43) et la deuxième roue de livraison (33 ; 53) comprennent chacune au moins une indentation (233) et au moins un orifice d'aspiration (231), la première roue de ligne de pliage (25 ; 451) et la deuxième roue de ligne de pliage (35 ; 551) comprennent chacune au moins une protubérance (251) et au moins un orifice d'aspiration (253), et les protubérances de la première roue de ligne de pliage et de la deuxième roue de ligne de pliage et les indentations de la première roue de livraison et de la deuxième roue de livraison sont agencées de sorte que, en utilisation, lorsque les roues tournent, les protubérances des première et deuxième roues de ligne de pliage rencontrent respectivement les indentations des première et deuxième roues de livraison pour réaliser les premières lignes de pliage respectives sur le premier produit en fibres porté par la première roue de livraison et sur le deuxième produit en fibres porté par la deuxième roue de livraison.

2. Machine à plier selon la revendication 1, dans laquelle un périmètre de la première roue de livraison (23 ; 43) est supérieur à un périmètre de la première roue de pliage (29 ; 49), et un périmètre de la deuxième roue de livraison (33 ; 53) est supérieur à un périmètre de la deuxième roue de pliage (39).

3. Machine à plier selon la revendication 2, dans laquelle le périmètre de la première roue de pliage (29 ; 49) est égal aux deux tiers du périmètre de la première roue de livraison (23 ; 43), et le périmètre de la deuxième roue de pliage est égal aux deux tiers du périmètre de la deuxième roue de livraison (33 ; 59).

4. Machine à plier selon l'une quelconque des revendications précédentes, dans laquelle le premier produit en fibres (22) et le deuxième produit en fibres (32) comprennent chacun une extrémité avant (225 ; 325) et une extrémité arrière (227 ; 327), une direction allant de l'extrémité arrière vers l'extrémité avant est une direction de livraison du premier produit en fibres et du deuxième produit en fibres, la première roue de livraison (23 ; 43) et la première roue de ligne de pliage (25) sont agencées pour réaliser la première ligne de pliage (221) sur le premier produit en fibres à un tiers de la longueur du premier produit en fibres distant de l'extrémité avant (227), et la deuxième roue de livraison (33 ; 53) et la deuxième roue de ligne de pliage sont agencées pour réaliser la première ligne de pliage (321) sur le deuxième produit en fibres à un tiers de la longueur du deuxième produit en fibres distant de l'extrémité avant (325).

5. Machine à plier selon la revendication 4, dans laquelle la première roue de pliage (29 ; 49) et la deuxième roue de pliage (39 ; 53) sont agencées pour réaliser la deuxième ligne de pliage sur le premier produit en fibres et le deuxième produit en fibres à un tiers de la longueur du premier produit en fibres et du deuxième produit en fibres distant des extrémités arrière respectives (227, 372) des premier et deuxièmes produits en fibres.

6. Machine à plier selon l'une quelconque des revendications précédentes, dans laquelle chacune de la première roue de pliage et de la deuxième roue de pliage comprend au moins une protubérance et au moins une indentation, et la protubérance (291) et l'indentation (293) de la première roue de pliage (29) se conforment respectivement à l'indentation (393) et à la protubérance (391) de la deuxième roue de pliage (39) pour réaliser la deuxième ligne de pliage sur le premier produit en fibres et le deuxième produit en fibres.

7. Machine à plier selon l'une quelconque des revendications précédentes, dans laquelle le premier dispositif de coupe (21 ; 41) comprend un premier couteau de coupe (213) et une première roue de coupe (211), le premier couteau de coupe et la première roue de coupe étant adjacents et agencés pour couper le premier produit en fibres passant entre eux, et le deuxième dispositif de coupe (31 ; 51) comprend un deuxième couteau de coupe et une deuxième roue de coupe, le deuxième couteau de coupe et la deuxième roue de coupe étant adjacents et agencés pour couper le deuxième produit en fibres passant entre eux.

8. Machine à plier selon l'une quelconque des revendications précédentes, comprenant en outre au moins une troisième roue de ligne de pliage (453), au moins une troisième roue à platine (473), au moins une quatrième roue de ligne de pliage (553) et au moins une quatrième roue à platine (573), dans laquelle la troisième roue de ligne de pliage et la troisième roue à platine sont disposées entre le premier dispositif de coupe (41) et la première roue de ligne de pliage (451) pour plier le premier produit en fibres en deux moitiés, et la quatrième roue de ligne de pliage et la quatrième roue à platine sont disposées entre le deuxième dispositif de coupe (51) et la deuxième roue de ligne de pliage (551) pour plier le deuxième produit en fibres en deux moitiés.
